# EUROPEAN PATENT APPLICATION

(11) **EP 3 693 024 A1**
(43) Date of publication of application: **12.08.2020**
(21) Application number: 19155556.4
(22) Date of filing: 05.02.2019
(51) Int. Cl.: A61K 47/69, B82Y 15/00, A61K 48/00, G01N 1/30

(54) **CONTROL FOR NUCLEIC ACID PREPARATION AND/OR DETECTION METHODS**

(71) Applicant: Altona Diagnostics GmbH, 22767 Hamburg (DE)
(72) Inventor: Friedrichsen, Sönke, 22529 Hamburg (DE); Kolkenbrock, Stephan, 22549 Hamburg (DE)
(74) Representative: Kuttenkeuler, David

(57) **Abstract**

The present invention belongs to the field of nucleic acid detection, particularly in-vitro diagnostics. The invention concerns amongst others the amplification of at least one or more target nucleic acid that may be present in at least one sample using an inventive control, which comprises a particle comprising a polycationic compound and a nucleic acid. The present invention relates also to uses of the inventive controls, methods for the preparation, diagnostic tools and kits.

## Description

### Technical field

The present invention belongs to the field of nucleic acid detection, particularly in-vitro diagnostics. The invention concerns amongst others the amplification of at least one or more target nucleic acid that may be present in at least one sample using an inventive control, which comprises a particle comprising a polycationic compound and a nucleic acid. The present invention relates also to uses of the inventive controls, methods for the preparation, diagnostic tools and kits.

### Background of the invention

Molecular diagnostics based on the detection of nucleic acids that are suspected to be present in a whole variety of sources relies heavily on nucleic acid amplification. Examples for diagnostic applications of nucleic acid amplification and detection are, for example, the detection of infectious agents such as viruses, bacteria, fungi or parasites, of oncology markers or any other markers of interest that are indicative of the physiological status of the source organism (e.g., pregnancy, hereditary diseases, etc.).

The best-known amplification technique is Polymerase Chain Reaction (PCR). Other amplification reactions comprise, inter alia, the Ligase Chain Reaction, Polymerase Ligase Chain Reaction, Gap-LCR, Repair Chain Reaction, 3SR, NASBA, Strand Displacement Amplification (SDA), Transcription Mediated Amplification (TMA), RCA (rolling circle amplification) und RPA (recombinase polymerase ampflification), HDA (Helicase depending amplification), isothermal amplification, and Qd-amplification. In the meantime, systems for PCR-based analysis have been automated and frequently allow for the real-time detection of product amplification during the PCR process in the same reaction vessel are also known in the art.

While the sensitivity and speed of detection of nucleic acid detection methods has increased considerably, particularly in diagnostic methods, the quality of these methods must be tightly controlled to avoid false results with potentially life-threatening consequences. Controls are used as standards to rule out that one or more of the steps of the nucleic acid detection methods did not function properly, for example, due to contaminations with nucleases, low amounts of nucleic acids obtained during extraction and/or purification from a given sample, or due to low-quality reagents used in these detection methods.

Frequently, nucleic acid controls at a known quantity, which have a known nucleic acid sequence which is at least partially different from the target sequences that should be detected are "spiked in" the reaction mixtures. If these sequences are used as positive controls the nucleic acid sequences may also be identical to a target sequence. The reaction mixtures may be samples of interest, such as clinical samples, or they may be real control samples, e.g. those comprising an artificial sample medium plus the nucleic acid control (positive control reaction). The detection and analysis of the control sequences ensures that the various steps of the respective methods functioned properly. At the same time, it is possible, of course, to perform negative control reactions in separate reaction vessels that may contain no nucleic acids. In these negative reactions no target nucleic acids and/or control nucleic acids should be detectable, which would be a sign for absence of contamination (spill over).

A number of controls for use in nucleic acid detection methods are known. For example, naked DNA having a different nucleic acid sequence than the target nucleic acid may be added to the reactions. Further, there have been attempts to add 'packaged' control nucleic acids to the reaction mixtures, e.g. viral particles, such as plant viruses (Tobacco Mosaic Virus, TMV), lentiviruses, or liposomes containing unrelated ('non-target') nucleic acids. Each one of these methods has advantages and disadvantages such as the sensitivity of naked DNA, the ease or difficulties of preparation of 'packaged' controls, the compatibility of the added matter with other components that are present in the reaction mixtures, the quantification of the amount of nucleic acids which are used as control materials, the risk of infectivity associated with the use of viral particles, et cetera.

Therefore, there is a need for easily producible, reliable controls that are preferably practically inert vis-à-vis other components of the reaction mixtures. These controls preferably should be quantifiable, they should be suitable for storage for an extended period of time, ideally for several months, under a variety of conditions (e.g., at different temperatures) and essentially without signs of degradation over a desirable period of time. They should be suitable for lyophilization and rehydration. These are some of the requirements that a control in nucleic acid detection methods should have.

The controls for nucleic acid detection provided herein fulfill all of the above desirable properties as discussed in more detail below. All documents discussed herein are herewith incorporated by reference in their entirety.

### Brief description of the Figures

Fig.1: Deacetylation of chitin (N-acetyl-D-glucosamine) to partially acetylated chitosan (mixture of N-acetyl-D-glucosamine and D-glucosamine). The deacetylation may occur chemically or enzymatically, whereas the latter method may generate a non-random pattern of acetylation (modified version from Wikimedia Commons, the free media repository).
Fig. 2: Different modes of interaction of nucleic acids with nanoparticles during preparation of the same (electrostatic interaction and encapsulation) and after preparation (adsorption) (figure modified from Mao SR, Sun W, Kissel T (2010) Adv. Drug Deliver. Rev. 62:12).
Fig. 3: Four different structures for nanosystems interacting with nucleic acids (Figure modified from Krauel K, Pitaksuteepong T, Davies N.M. et al. (2004) Am. J. Drug Deliv. 2: 251).
Fig. 4: Schematic presentation of chitosan nanoparticle generation by ionic gelation. (Copyright: Sruthi Sreekumar)
Fig. 5: Schematic presentation of chitosan nanocapsule generation by solvent displacement technique. (Copyright: Sruthi Sreekumar)

### Detailed description

The present invention relates to the following embodiments.

In a first embodiment, the invention relates to a control for nucleic acid preparation and/or detection methods comprising at least one polycationic particle and a nucleic acid.

In a further embodiment the invention relates to a control for nucleic acid preparation and/or detection methods according to the first embodiment, wherein the polycationic particle comprises a polycationic polysaccharide, and/or a polylysine, and/or a derivative of any of these.

In one embodiment the invention relates to a control for nucleic acid preparation and/or detection methods according to the first embodiment, wherein the polycationic particle comprises a polycationic polysaccharide, and/or a derivative thereof.

In one embodiment the invention relates to a control for nucleic acid preparation and/or detection methods according to the first embodiment, wherein the polycationic particle consists of a polycationic polysaccharide, and/or a derivative thereof.

In one embodiment the invention relates to a control for nucleic acid preparation and/or detection methods, wherein the polycationic particle comprises polylysine, and/or a derivative thereof.

In one embodiment the invention relates to a control for nucleic acid preparation and/or detection methods according to the first embodiment, wherein the polycationic particle consists of polylysine, and/or a derivative thereof.

In another embodiment the invention relates to a control for nucleic acid preparation and/or detection methods according to any of the foregoing embodiments, wherein polycationic polysaccharide comprises at least partially deacetylated N-acetyl-D-glucosamine (GlcNAc, A) residues. Hereinafter, the deacetylated N-acetyl-D-glucosamine residues are occasionally referred to as (GlnN, D) residues or as D-glucosamine. Co-polymers comprising at least partially deacetylated N-acetyl-D-glucosamine residues and GlnN, D are also referred to as "chitosan".

In another embodiment the invention relates to a control for nucleic acid preparation and/or detection methods according to any of the foregoing embodiments, wherein the polycationic polysaccharide comprises co-polymers of N-acetyl-D-glucosamine (GlcNAc, A) residues and D-glucosamine residues (GlcN, D), also known as "chitosan".

In another embodiment the invention relates to a control for nucleic acid preparation and/or detection methods according to any of the foregoing embodiments, wherein the polycationic polysaccharide consists of deacetylated N-acetyl-D-glucosamine (GlcNAc, A) residues.

In one embodiment the invention relates to a control for nucleic acid preparation and/or detection methods according to the first embodiment, wherein the polycationic particle comprises the polysaccharide chitosan, and/or a derivative thereof. Derivatives may be chemically modified chitins, e.g. glycol-chitin, which in turn may be used to prepare glycol-chitosan (e.g., disclosed in US20120053331A1; reviewed in V. K.Mourya and N. N.Inamdar, Reactive and Functional Polymers, Volume 68, Issue 6, June 2008, Pages 1013-1051).

In another embodiment the invention relates to a control for nucleic acid preparation and/or detection methods according to any of the foregoing embodiments, wherein the nucleic acid is selected from the group comprising or consisting of DNA, RNA, cDNA, miRNA, siRNA, ssRNA, ssDNA, dsRNA and gRNA.

In another embodiment the invention relates to a control for nucleic acid preparation and/or detection methods according to any of the foregoing embodiments, wherein the nucleic acid is DNA.

In another embodiment the invention relates to a control for nucleic acid preparation and/or detection methods according to any of the foregoing embodiments, wherein the nucleic acid is RNA.

In another embodiment the invention relates to a control for nucleic acid preparation and/or detection methods according to any of the foregoing embodiments, wherein the polycationic polysaccharide consists of chitosans and wherein the nucleic acid is DNA or RNA.

In another embodiment the invention relates to a control for nucleic acid preparation and/or detection methods according to any of the foregoing embodiments, wherein the process comprises a nucleic acid extraction step.

In another embodiment the invention relates to a control for nucleic acid preparation and/or detection methods according to any of the foregoing embodiments, wherein the process comprises a nucleic acid amplification step.

In another embodiment the invention relates to a control for nucleic acid preparation and/or detection methods according to any of the foregoing embodiments, wherein the process comprises a nucleic acid detection step.

In another embodiment the invention relates to a control for nucleic acid preparation and/or detection methods according to any of the foregoing embodiments, wherein the process comprises a nucleic acid extraction step and nucleic acid amplification step.

In another embodiment the invention relates to a control for nucleic acid preparation and/or detection methods according to according to any of the foregoing embodiments, wherein the process comprises a nucleic acid extraction step and nucleic acid amplification step and a nucleic acid detection step.

In another embodiment the invention relates to a control for nucleic acid preparation and/or detection methods according to any of the foregoing embodiments, wherein the polycationic polysaccharide and the nucleic acid form a nanoparticle and/or a nanosystem, optionally a nanosphere and/or a nanocapsule.

In another embodiment the invention relates to a control for nucleic acid preparation and/or detection methods according to any of the foregoing embodiments, wherein the polycationic polysaccharide and the nucleic acid form a nanoparticle and/or a nanosystem, optionally a nanosphere and/or a nanocapsule, wherein the nucleic acid is DNA or wherein the nucleic acid is RNA, wherein polycationic polysaccharide comprises at least partially deacetylated N-acetyl-D-glucosamine (GlcNAc, A) residues, wherein the at least partially deacetylated N-acetyl-D-glucosamine (GlcNAc, A) residues comprise co-polymers of GlcNAc and D-glucosamine (GlcN, D), i.e., chitosan and/or derivatives thereof. In another embodiment the co-polymers consist of chitosan.

In another embodiment the invention relates to a control for nucleic acid preparation and/or detection methods according to any of the foregoing embodiments, wherein the polycationic polysaccharide and the nucleic acid are present in form of an adsorbed nanoparticle, an adsorbed nanosystem an adsorbed nanosphere or in form of an adsorbed nanocapsule, or wherein the nucleic acid is present in a polycationic polysaccharide nanoparticle, a polycationic polysaccharide nanosystem an adsorbed polycationic polysaccharide nanosphere or in form of an adsorbed polycationic polysaccharide nanocapsule.

In another embodiment the invention relates to a control for nucleic acid preparation and/or detection methods according to any of the foregoing embodiments, wherein the nucleic acid is a synthetically produced nucleic acid and/or a nucleic acid obtained from a natural source.

In another embodiment the invention relates to a control for nucleic acid preparation and/or detection methods according to any of the foregoing embodiments, wherein the nucleic acid is a synthetically produced nucleic acid or the nucleic acid is obtainable from a natural source selected from the group comprising nucleic acids derivable for example from a prokaryote, an eukaryote, an archaea, a microorganism, a bacteria, a virus, a bacteriophage, a fungus, a protozoa, a parasite, a mollusk, , an arthropod, an insect, a mammal, a bird, a fish, a reptile, an amphibian, a human, a plant, or is an in vitro produced fragment thereof, or a nucleic acid that does not exist in nature.

In another embodiment the invention relates to a use of the control for nucleic acid preparation and/or detection methods according to any of the foregoing embodiments in a nucleic acid detection method.

In another embodiment the invention relates to a use of the control for nucleic acid preparation and/or detection methods according to any of the foregoing embodiments in a diagnostic or in a forensic method or in a step of a diagnostic or a forensic method.

In another embodiment the invention relates to a use of the control for nucleic acid preparation and/or detection methods according to any of the foregoing embodiments in a diagnostic method or in a step of a diagnostic method.

In another embodiment the invention relates to a use of the control for nucleic acid preparation and/or detection methods according to any of the foregoing embodiments in a forensic method or in a step of a forensic method.

In another embodiment the invention relates to a use of the control for nucleic acid preparation and/or detection methods according to any of the foregoing embodiments, wherein said nucleic acid detection method, said diagnostic method, said forensic method, or said step of said diagnostic method or said forensic method comprises the detection and/or quantification of a nucleic acid in a sample, wherein said nucleic acid is a nucleic acid derived from an organism/cell from which the sample was obtained, or the nucleic acid of synthetic origin, or the nucleic acid is present in an extracorporeal environment.

In another embodiment the invention relates to a use of the control for nucleic acid preparation and/or detection methods according to any of the foregoing embodiments wherein the nucleic acid is present in an extracorporeal environment and is indicative of the presence of an organism and / or a defined sequence of interest, like for example an infectious agent, a parasite, a protozoa, a toxic organism, a microorganism, a bacterium, a virus, a fungus, a parasite, an archaea, an eukaryote or any defined genetic marker.

In another embodiment the invention relates to a use of the control for nucleic acid preparation and/or detection methods according to any of the foregoing embodiments wherein the nucleic acid is present in an extracorporeal environment and is indicative of the presence of a bacterium, particularly of a pathogenic bacterium.

In another embodiment the invention relates to a use of the control for nucleic acid preparation and/or detection methods according to any of the foregoing embodiments wherein the nucleic acid is present in an extracorporeal environment and is indicative of the presence of a virus, particularly of a pathogenic virus.

In another embodiment the invention relates to a use of the control for nucleic acid preparation and/or detection methods according to any of the foregoing embodiments wherein the nucleic acid is present in an extracorporeal environment and is indicative of the presence of a fungus, particularly of a pathogenic fungus.

In another embodiment the invention relates to a use of the control for nucleic acid preparation and/or detection methods according to any of the foregoing embodiments wherein the nucleic acid is present in an extracorporeal environment and is indicative of the presence a protozoa, particularly of a pathogenic protozoa.

In another embodiment the invention relates to a use of the control for nucleic acid preparation and/or detection methods according to any of the foregoing embodiments wherein the nucleic acid is present in an extracorporeal environment and is indicative of the presence a parasite, particularly of a pathogenic parasite.

In another embodiment the invention relates to a use of the control for nucleic acid preparation and/or detection methods according to any of the foregoing embodiments, wherein the nucleic acid is present in an extracorporeal environment and the method is a forensic method.

In another embodiment the invention relates to a chemical formulation or a composition comprising a control according to any of the foregoing embodiments.

In another embodiment the invention relates to a formulation or composition according to any of the foregoing embodiments, wherein said formulation or said composition further comprises a solvent.

In another embodiment the invention relates to a formulation or composition according to any of the foregoing embodiments, wherein said formulation or said composition further comprises a nuclease inhibitor.

In another embodiment the invention relates to a formulation or composition according to any of the foregoing embodiments, wherein said formulation or said composition further comprises a preservative.

In another embodiment the invention relates to a formulation or composition according to any of the foregoing embodiments relating to formulations/compositions, wherein said formulation or composition is in a lyophilized state.

In another embodiment the invention relates to any one of the above described controls comprising at least one polycationic particle and a nucleic acid, wherein said control is in a lyophilized state. The lyophized controls according to the present invention are more stable to external influences and may be reconstituted with a suitable medium as needed. The lyophilized controls according to the present invention may also be stored and/or transported in a frozen state to maximize their stability. The lyophilized and/or frozen controls according to the invention may be part of a kit of parts comprising containers or vessels for reagents such as the control, media for reconstitution, buffers, oligonucleotide primers and/or probes, nucleotides or nucleosides as building blocks for nucleic acid amplification reactions, instructions for use, control reagents, marker molecules that enhance detectability (e.g., fluorescent dyes, or other detectable labels).

In another embodiment the invention relates to a method for the detection of a target nucleic acid, comprising either the step of adding the control according to any of the foregoing embodiments relating to controls, or the step of adding a formulation or a composition according to any of the appropriate foregoing embodiments relating to such formulation and/or composition to a sample.

In another embodiment the invention relates to a method according to the preceding embodiment, wherein the sample is selected from the group comprising a clinical sample, an environmental sample, a forensic sample, an internal control sample.

In another embodiment the invention relates to a method according to the preceding embodiments, wherein the sample is a clinical sample.

In another embodiment the invention relates to a method according to the preceding embodiment, wherein the sample is an environmental sample.

In another embodiment the invention relates to a method according to the preceding embodiment, wherein the sample is a forensic sample.

In another embodiment the invention relates to a method according to the preceding embodiment, wherein the sample is an internal control sample.

In another embodiment the invention relates to a method according to any of the foregoing embodiments, wherein the clinical sample is selected from the group comprising plasma, serum, urine, stool, whole blood, CSF, saliva, respiratory tract-derived samples, skin swabs, solid tissues, tumors, biopsy materials, and/or hair, etc.

In another embodiment the invention relates to a method according to any of the foregoing embodiments, wherein the clinical sample is formalin-fixed paraffin-embedded tissue (FFPE-tissue).

In another embodiment the invention relates to a method according to any of the foregoing embodiments, wherein the control according to any of the respective foregoing embodiments, or the formulation or composition according to any of the respective foregoing embodiments, is added to (spiked into) the sample prior to extraction of nucleic acids suspected to be present in said sample.

In another embodiment the invention relates to a method according to any of the foregoing respective embodiments, wherein the method is a diagnostic method.

In another embodiment the invention relates to a method according to any of the foregoing respective embodiments, wherein said method may comprise the following steps:
a) adding the control according to any one of the appropriate embodiments or a formulation or a composition according to anyone of the appropriate embodiments to a sample to form a mixture,
b) obtaining nucleic acids from the mixture obtained in step a),
c) performing an amplification reaction using oligonucleotides specifically hybridizing with at least one target nucleic acid and/or oligonucleotides specifically hybridizing with the control nucleic acid,
d) measuring / detecting the amplification product obtained in step c).

In another embodiment the invention relates to a diagnostic kit (e.g. a kit of parts, which may be packed into a container, such as a cardboard box or a plastic box) comprising at least one reagent for the sampling of nucleic acids and/or for the extraction of nucleic acids and/or for the amplification of nucleic acids and/or for the detection of nucleic acids, and a control or a formulation or a composition comprising a control according to any of the respective foregoing embodiments, and optionally instructions for use.

In another embodiment the invention relates to a point-of-care device comprising a control according to the present invention.

In another embodiment the invention relates to a point-of-care device comprising a control according to the present invention, wherein the device may take any possible form or shape provided it is suitable to be used in a method as defined above, i.e. it should comprise an opening for the addition of a given sample and physicochemical means to execute the methods, e.g. it may take the form of a cartridge or a disc and conventionally comprises chemical reagents required for the indicated purpose, e.g., enzymes, primers, probes, nucleotides and, in accordance with the invention, a least one control described in any of the foregoing embodiments. Point-of-care devices according to the invention may contain the inventive controls in a separate chamber, in a flow path, or in a compartment comprising a buffer.

In another embodiment the invention relates to a method of producing the control according to any one of the respective preceding embodiments, said method comprising the steps:
a) Admixing a solubilized polycationic compound as defined in any one of the appropriate foregoing embodiments and at least one nucleic acid molecule,
b) Provision of at least one nanoparticle and/or nanosphere as defined in the appropriate foregoing embodiments,
c) Optionally lyophilizing the obtained nanoparticle and/or nanosphere provided in step b)
d) Optionally storing the nanoparticle and/or nanosphere,
e) Further optionally subjecting the nanoparticle and/or nanosphere to qualitative analysis.

### Summary of embodiments

1. A control for nucleic acid preparation and/or detection methods comprising at least one polycationic particle and a nucleic acid.
2. The control for nucleic acid preparation and/or detection methods according to embodiment 1, wherein the polycationic particle comprises a polycationic polysaccharide, a polycationic amino acid, particularly polylysine, or derivative of any of these.
3. The control for nucleic acid preparation and/or detection methods according to embodiments 1 or 2, wherein polycationic polysaccharide comprises at least partially deacetylated N-acetyl-D-glucosamine (GlcNAc, A) residues and/or a derivative thereof.
4. The control for nucleic acid preparation and/or detection methods according to any one of the preceding embodiments 1 to 3, wherein the polycationic polysaccharide comprises at least partically deacetylated N-acetyl-D-glucosamine (GlcNAc, A) residues co-polymerized with D-glucosamine (GlcN, D) and/or a derivative thereof.
5. The control for nucleic acid preparation and/or detection methods according to any one of the preceding embodiments 1 to 4, wherein the polycationic polysaccharide comprises chitosan and/or a derivative thereof.
6. The control for nucleic acid preparation and/or detection methods according to any one of the preceding embodiments 1 to 5, wherein the nucleic acid is selected from the group comprising DNA, RNA, cDNA, siRNA, ssRNA, ssDNA, dRNA, gRNA.
7. The control for nucleic acid preparation and/or detection methods according to any one of the preceding embodiments 1 to 6, wherein the polycationic polysaccharide and the nucleic acid form a nanoparticle and/or a nanosystem, optionally a nanosphere and/or a nanocapsule.
8. The control for nucleic acid preparation and/or detection methods according to any one of the preceding embodiments 1 to 7, wherein the polycationic polysaccharide and the nucleic acid are present in form of an adsorbed nanoparticle, an adsorbed nanosystem an adsorbed nanosphere or in form of an adsorbed nanocapsule, or wherein the nucleic acid is present in a polycationic polysaccharide nanoparticle, a polycationic polysaccharide nanosystem an adsorbed polycationic polysaccharide nanosphere or in form of an adsorbed polycationic polysaccharide nanocapsule.
9. The control for nucleic acid preparation and/or detection methods according to any one of the preceding embodiments 1 to 8, wherein the nucleic acid is a synthetically produced nucleic acid or a nucleic acid obtained from a natural source.
10. The control for nucleic acid preparation and/or detection methods according to any one of the preceding embodiments 1 to 9, wherein the nucleic acid is a synthetically produced nucleic acid or the nucleic acid is obtainable from a natural source selected from the group comprising nucleic acids derivable from a microorganism, a bacteria, a virus, a fungus, a parasite, an archaea, a eukaryote, a mammal, a bird, a fish, a human, an insect, a plant, or is an in vitro produced fragment thereof, or a nucleic acid that does not exist in nature.
11. Use of the control for nucleic acid preparation and/or detection methods according to any of the preceding embodiments 1 to 10, in a nucleic acid detection method.
12. Use according to embodiment 11, wherein the nucleic acid detection method comprises a nucleic acid extraction step.
13. Use according to embodiments 11 and/or 12, wherein the nucleic acid detection method comprises a nucleic acid amplification step.
14. Use according to any one of embodiments 11 to 13, wherein the nucleic acid detection method comprises a nucleic acid detection step.
15. Use according to any one of embodiments 11 to 14, wherein the nucleic acid detection method is a diagnostic or in a forensic method or in a step of a diagnostic or a forensic method.
16. Use according to any of the preceding embodiments 1 to 15, wherein said nucleic acid detection method, said diagnostic method, said forensic method, or said step of said diagnostic method or said forensic method comprises the detection and/or quantification of a nucleic acid in a sample, wherein said nucleic acid is a nucleic acid derived from an organism/cell from which the sample was obtained, or the nucleic acid is of synthetic origin, or the nucleic acid is present in an extracorporeal environment.
17. Use according to any of the preceding embodiments 1 to 16, wherein the nucleic acid is present in an extracorporeal environment and is indicative of the presence of an organism of interest, an infectious agent, a parasite, a toxic organism, from a microorganism, a bacteria, a virus, a fungus, a parasite, an archaea, or an eukaryote.
18. Use according to any of the preceding embodiments 1 to 17, wherein the nucleic acid is present in an extracorporeal environment and the method is a forensic method.
19. A formulation or a composition comprising a control according to anyone of the preceding embodiments 1 to 10.
20. The formulation or composition according to any one of the preceding embodiment 19, wherein said formulation or said composition further comprises a solvent.
21. The formulation or composition according to any one of the preceding embodiments 19 or 20, wherein said formulation or said composition further comprises a nuclease inhibitor.
22. The formulation or composition according to any one of the embodiments claims 19 to 21, wherein said formulation or said composition further comprises a preservative.
23. The formulation or composition according to any one of embodiments 19 to 22, or the control according to any one of claims 1 to 10, wherein said formulation or composition or said control is in a lyophilized state.
24. A method for the detection of a target nucleic acid, comprising the step of adding the control according to anyone of embodiments 1 to 10, or a formulation or a composition according to anyone of the preceding embodiments 19 to 23 to a sample.
25. The method according to embodiment 24, wherein the sample is a clinical sample, an environmental sample, a forensic sample, an internal control sample.
26. The method according to any one of embodiments 24 or 25, wherein the clinical sample is selected from the group comprising plasma, serum, urine, stool, whole blood, cerebrospinal fluid, saliva, respiratory tract-derived samples, skin swabs, solid tissues, tumors, biopsy materials, and/or hair.
27. The method according to any one of embodiments 24 to 26, wherein the control according to anyone of the preceding embodiments 1 to 10, or the formulation or composition according to anyone of the preceding embodiments 19 to 23 is added to the sample prior to extraction of nucleic acids suspected to be present in said sample.
28. The method according to any one of embodiments 24 to 27, wherein the method is a diagnostic method.
29. The method according to any one of embodiments 24 to 28, wherein said method comprises the following steps:
   a) adding the control according to any one of embodiments 1 to 10 or a formulation or a composition according to anyone of embodiments 19 to 23 to a sample to form a mixture,
   b) obtaining nucleic acids from the mixture obtained in step a),
   c) performing an amplification reaction using oligonucleotides specifically hybridizing with at least one target nucleic acid and/or oligonucleotides specifically hybridizing with the control nucleic acid,
   d) measuring / detecting the amplification product obtained in step c).
30. A kit of parts comprising at least one reagent for the sampling of nucleic acids and/or for the extraction of nucleic acids and/or for the amplification of nucleic acids and/or for the detection of nucleic acids, and a control or a formulation or a composition comprising a control according to any of the preceding embodiments 1 to 10 and/or 19 to 23, and optionally instructions for use.
31. A method of producing a control according to any one of the embodiments claims 1 to 10, said method comprising the steps:
   a) admixing a solubilized polycationic compound as defined in embodiments 1 to 10 and at least one nucleic acid molecule,
   b) provision of at least one nanoparticle and/or nanosphere as defined in the foregoing claims,
   c) optionally lyophilizing the obtained nanoparticle and/or nanosphere provided in step b)
   d) optionally storing the nanoparticle and/or nanosphere,
   e) further optionally subjecting the nanoparticle and/or nanosphere to qualitative analysis.
32. A method of producing a formulation or composition according to embodiments 19 to 23 comprising a control according to any one of the preceding claims 1 to 10, said method comprising the steps:
   a) admixing a solubilized polycationic compound as defined in the foregoing claims and at least one nucleic acid molecule,
   b) provision of at least one nanoparticle and/or nanosphere as defined in the foregoing claims,
   c) admixing the nanoparticle and/or nanosphere as defined in step b) with at least one chemical excipient to form a formulation or composition,
   d) optionally lyophilizing the obtained formulation or composition provided in step c).
33. A point-of-care device comprising a control for nucleic acid preparation and/or detection methods according to any of the preceding embodiments 1 to 10, or a formulation or composition according to any one of embodiments 19 to 23.

### Definitions

In some embodiments of the present invention, the sample to be analyzed serves as an in-process control. This means that the sample to be analyzed comprises (preferably at a known quantity) a target nucleic acid in a medium, e.g., as artificial plasma, stool, CSF, and the like. The internal control may of course also contain the target nucleic acid in natural medium, e.g., real plasma, stool, CSF, etc..

In some embodiments the control, which comprises particles comprising a polycationic compound, e.g., a polysaccharide such as defined throughout the description and the claims appended to this disclosure and nucleic acid is spiked into the in-process medium.

Subsequent steps of the detection methods as defined herein may therefore be performed using the medium comprising the spiked-in control. These steps may comprise at least one step selected from nucleic acid extraction/purification, nucleic acid transcription, nucleic acid amplification, measuring the amplified amount of nucleic acids, e.g., using fluorescent probes, etc. When the nucleic acids derived from spiked-in controls can be detected, the control will be considered as a positive control indicating that the detection method has worked correctly. On the other hand, when the nucleic acids derived from spiked-in controls are not detected, the control cannot be considered as a positive control indicating that the detection method has worked correctly.

As used herein, the term "control" comprises at least one nucleic acid and one polycationic compound interacting with said nucleic acid, such that the nucleic acid forms a complex with said polycationic compound. These controls may be used, e.g., in the detection of nucleic acids in a sample, and are suitable for determination that the steps of such methods have functioned correctly. The controls may also be used both, quantitatively and qualitatively, i.e. the presence or absence as well as the amount of a given nucleic acid ("nucleic acid target") may be measured.

As used herein, the term "nucleic acid" (hereinafter also referred to as "NA") means an assembly of nucleotides, wherein said assembly comprises a chain, or a strand, of nucleotides forming a linear oligomer or polymer of DNA and/or RNA. DNA and RNA may be single- or double-stranded, naturally occurring or not naturally occurring as known in the art, with catalytic or non-catalytic activity, regulatory or non-regulatory elements. Nucleic acids may contain nucleotides different from adenine, thymine, cytosine, guanine or uracil, or are derivatives thereof.

As used herein, the term "polycationic" means an assembly of positive charges in an oligomer or homo- or heteropolymer originating from the positive charges of at least one subunit of said oligomer or homo- or heteropolymer, used herein as a compound for the production of positively charged particles, particularly, nanoparticles, wherein the amount of positive charge of the polymer increases with the length of the polymer. Such a polycationic polymer, in the context of the invention, may be an assembly of positively charged glycans, thus forming a positively charged polysaccharide and a positively charged nanoparticle. It is also possible that the nanoparticles of the present invention comprise a mixture of positively charged polysaccharide, e.g., glycans in a heteropolymer. Said positive charges may arise from the protonation of chemical groups, such as amino groups in monosaccharides, and thus can be pH-dependent.

As used herein, the term "polysaccharide" means a linear or branched assembly of monosaccharides, or glycans, through the formation of covalent glycosidic bonds between these monosaccharides, particularly a polymer of positively charged monosaccharides, more particularly a linear polymer comprising positively charged monosaccharides, even more particularly a linear polymer comprising positively charged monosaccharides, wherein the monosaccharide is a deacetylated N-acetyl-D-glucosamine.

As used herein, the term "N-acetyl-D-glucosamine " (GlcNAc, A) means a monosaccharide derivative of glucose, wherein the hydroxyl group on carbon number two of glucose is replaced with an amino group, which in turn has one hydrogen substituted with one acetyl group on its nitrogen atom, and stereoisomers thereof comprising N-acetyl-D-glucosamine.

As used herein, the term "deacetylated D-glucosamine" (GlcN, D) means a monosaccharide derivative of glucose, wherein the hydroxyl group on carbon number two of glucose is replaced with an amino group, and wherein said deacetylated D-glucosamine is also obtainable by deacetylation of N-acetyl-D-glucosamine.

As used herein, the term "chitosan" means a linear polymer of randomly distributed N-acetyl-D-glucosamine and deacetylated N-acetyl-D-glucosamine, linked via β-1,4 glycosidic bonds, wherein chitosan is obtainable by deacetylation of chitin. Alternatively, it is contemplated to use non-randomly distributed enzymatically produced chitosan.

As used herein, the terms "extraction", "isolation", "purification" of a nucleic acid mean the following: "Extraction" refers to the extraction of nucleic acid from any given source, e.g., a cell, tissue or organism, or cell-free material, by means of a scientific method. In the process of obtaining nucleic acid from a source, the term "extraction" refers also to the "isolation" and "purification". "Isolation" and "purification" refer to the process of separation of the nucleic acid of interest from most other components of a sample.

As used herein, the term "amplification" means a process wherein at least a portion of a nucleic acid molecule, referred to as a template, is complementarily copied into at least one additional nucleic acid molecule. In some embodiments, amplification includes a template-dependent in vitro enzyme-catalyzed reaction producing at least one copy of the nucleic acid molecule or at least one copy of a nucleic acid sequence that is partially complementary to said nucleic acid molecule. Amplification comprises replication of a nucleic acid molecule, and is performed using either isothermal or non-isothermal conditions as known to a person skilled in the art. As known in the art, RPA (Recombinase Polymerase Amplification) may also require the use of a recombinase (uvsX), a loading factor (uvsY), T4 SSB protein and a creatine kinase.

As used herein, the term "detection" means the action or process of identifying the presence of a specific nucleic acid in a sample, for example, by means of an amplification reaction that is catalyzed by an enzyme, e.g. polymerase chain reaction (PCR).

As used herein, the term "particle" refers to either a viral particle, or a small control as defined above, e.g., a nanoparticle, wherein a nanoparticle forms part of the subject matter of the invention and is defined further below.

The term "nanoparticle" means in general a synthetic particle with an average diameter in the order of magnitude of one nanometer or more, wherein the average diameter of a nanoparticle approximately ranges from 1 nm to 500 nm. Within the context of the subject matter of this invention, we refer to such particles as particles consisting of a polymer or a combination of polymers, comprising polysaccharide or polylysine particles, wherein said nanoparticles are either spherical, non-spherical, or tubular nanoparticles, and wherein the particle average diameter is between 10 to 1000 nm, for example 50 to 750 nm, particularly 100 to 500, more particularly 100 to 400 nm or 100 to 300 nm. In a preferred embodiment, said nanoparticles are positively charged and capable of non-covalently binding to charged molecules, wherein said charged molecules are, for example, nucleic acids.

As used herein, the term "nanosphere" means a nanoparticle as herein defined, wherein said nanoparticle has a spherical shape, and wherein the entirety of said nanoparticle consists of a polymer, or a combination of polymers, for example, a positively charged polymer or a combination of polymers. In some embodiments, said polymers are polysaccharides comprising chitosan, or a said polymer is a polylysine. In a specific embodiment, said nanospheres are positively charged and capable of adsorbing negatively charged molecules at the surface, wherein said charged molecules may be nucleic acids.

As used herein, the term "nanocapsule" means a nanoparticle as herein described, wherein said nanocapsule comprises an outer layer comprising polymer, and an inner core comprising a phase distinct from the polymer, in a preferred embodiment a liquid phase. In one embodiment, the outer layer of said nanocapsules is positively charged, whereas the inner layer is negatively charged. The positively charged outer layer is capable of adsorbing negatively charged molecules, e.g., nucleic acids, thereby being capable of binding nucleic acid at the surface or encapsulating nucleic acids.

As used herein, the term "completely or partially deacetylated" means partially devoid or completely devoid of an acetyl group, wherein the compound that is partially devoid of acetylation comprises a polysaccharide, preferably a chitosan molecule or polymer, or a solution containing said chitosan molecule or polymer, or nanoparticles formed therefrom, and wherein partially devoid means a degree of acetylation between zero and 50%, more particulary 0 to 30%. The percentage, or degree of deacetylation, or acetylation, in the case of a chitosan-based compound as herein described, refers to the average degree of acetylation of all chitosan molecules in a particular sample, and specifically relates to the deacetylation of an amino group of an N-acetyl-D-glucosamine moiety.

As used herein, the terms "synthetically produced NA" or "synthetic NA" or similar terms such as "artificially produced NA" or "non-natural NA" or "NA that does not exist in nature" or "nucleic acid obtained from a natural source" are defined as follows. Synthetically produced, synthetic, artificially produced, non-natural nucleic acids, or nucleic acids that do not exist in nature, are nucleic acids that are not directly obtainable from a natural source by means of, for example, extraction or isolation of nucleic acid.

As used herein, the term "adsorbed on a nanocapsule" or "adsorbed on a (nano-) particle" means the nucleic acid is present on the surface of the capsule and that it is immobilised by static or a chemical interactions with the material of the capsule or (nano-) particle. This does not exclude the option that nucleic acids are both adsorbed to the surface of the capsule and at the same time nucleic acids are present within the capsule.

As used herein, the terms "obtained from" or "obtainable from" mean that a material of interest, for example a nucleic acid, is obtained from its environment by the process of extraction, purification and/or isolation as to find above. "Obtainable from" indicates that the material may be obtained from a given environment, for example using a particular process or method, but that also material that has been obtained by a different method or process is encompassed by this term.

As used herein, the term "microorganism" means that an organism is generally only visually identifiable upon appropriate enlargement, for example using a microscope. As used herein, the term microorganism covers bacteria, viruses, fungi, protozoa, parasites, et cetera, as defined throughout the disclosure and as commonly known in the art, for example in textbooks relating to microbiology. In particular embodiments of the present invention, the microorganisms are capable of infecting larger organisms or cells thereof as hosts and are the source of a disorder or disease, particularly pathogenic microorganisms, more particularly pathogenic microorganisms infecting animal, human and/or plant cells.

As used herein, the term "bacteria" refers to the commonly accepted meaning in the art. Particularly, some embodiments of the present invention relate to pathogenic bacteria, more particularly bacteria infecting animals, humans, and plants such as *Acetinobacter* spp., *Anaplasma spp., Bacillus spp., Bartonella spp., Bordetella spp., Borellia spp., Brucella spp., Burkholderia spp., Campylobacter spp., Chlamydia spp., Clostridium spp., Corynebacterium spp., Coxiella spp., Ehrlichia spp., Enterococcus spp., Enterobacter spp., Escherichia spp., Francisella spp., Haemophilus spp., Helicobacter spp., Klebsiella spp., Legionella spp., Leptospira spp., Listeria spp., Moraxella spp., Mycobacterium spp., Mycoplasma spp., Neisseria spp., Pasteurella spp., Pseudomonas spp., Rickettsia spp., Salmonella spp., Shigella spp., Spirochaeta spp., Staphylococcus spp., Streptococcus spp., Streptomyces spp., Treponema spp., Trichomonas spp., Ureoplasma spp., Vibrio spp. Yersinia spp.,* and other known bacteria.

As used herein, the term "virus" refers to the commonly accepted meaning in the art. Particularly, some embodiments of the present invention relate to animal viruses, more particularly viruses infecting animals, humans, and plants more particularly viruses that are the cause for diseases or disorders in animals, humans, and plants for example Adenoviruses, Arboviruses (e.g. Flaviviridae like: Alkhurma viruses, West Nile viruses, Dengue viruses, Yellow Fever viruses, Japan Encephalitis viruses, TBEV, Powassan viruses, Zika viruses; Togaviridae like: EEE viruses, WEE viruses, VEE viruses; CHIK viruses; Bunyaviridae like: CCHF viruses, RVF viruses, Toskana viruses, Heartland viruses, Oropouche viruses), Arenaviruses (e.g. Lassa viruses, Machupo viruses), Astroviruses, Bocaviruses, Coronaviruses, Enteroviruses, Filoviruses (e.g. Marburg viruses, Ebola viruses), Hantaviruses, Hendra viruses, Hepatitis viruses, Herpesviruses (e.g. Herpes simplex viruses 1 and 2, EBV, CMV, VZV, Human herpesviruses 6 and 7 and 8) Human Immunodeficiency viruses, Influenza viruses, Measles viruses, Metapneumoviruses, Mumps viruses, Nipah viruses, Noroviruses, Papillomaviruses, Parainfluenza viruses, Parechoviruses, Parvoviruses, Polyomaviruses (e.g. BK viruses, JC viruses), Poxviruses, Rabies Viruses, Respiratory Syncytial viruses, Rhinoviruses, Rotaviruses, Rubella viruses, Sapoviruses, Toroviruses, and other known viruses.

As used herein, the term "fungus" to the commonly accepted meaning in the art, and particularly *Aspergillus spp., Blastomyces spp., Coccidioides spp., Epidermophyton spp., Fusarium spp., Histoplasma spp., Microsporum spp., Pneumocystis spp., Sporothrix spp., Stachybotrus spp., Trichophyton spp.,* but also includes yeasts, particularly pathogenic yeasts like *Candida spp., Cryptococcus spp.,* and other known fungus.

As used herein, the term "protozoa" refers to the commonly accepted meaning in the art, e.g. *Acanthamoeba spp., Babesia spp., Blastocystis spp., Cryptosporidium spp., Cyclospora spp., Eimeria spp., Entamoeba spp., Enterobius spp., Giardia spp., Isospora spp., Leishmania spp., Microsporidium spp., Naegleria spp., Plasmodium spp., Sarcocystis spp., Toxoplasma spp., Trichomonas spp., Trypanosoma spp.,* and other known protozoa.

As used herein, the term "parasite" refers to the commonly accepted meaning in the art, e.g. Nematodes (e.g. *Ascaris spp., Enterobius spp., Strongyloides spp., Triuris spp*.); Filaria (e.g. *Brugia spp., Loa spp., Mansonella spp., Onchocerca spp., Wuchereria spp.*.); Cestodes (e.g. *Diphyllobothrium spp., Taenia spp*..); Trematodes (e.g. Fasciola spp., Schistosoma spp.,); and other known parasites.

As used herein, the term "detection" means that either the presence of a nucleic acid of interest as indicated above in the definition of the term "nucleic acid" or the microorganism from which such a nucleic acid is derived is revealed. According to the common understanding, this term implies that a detection method is used, for example a detection method based on PCR, which comprises various steps that permit to draw the conclusion that a given nucleic acid is present in a sample under investigation. Frequently, such methods include comparisons with positive or negative controls which may serve as qualitative and quantitative standards allowing the person analyzing said sample to draw the correct conclusion from a given measurement method, e.g. PCR, etc.

As used herein, the term "diagnosis" means that the presence of, for example given target nucleic acid in a sample indicates the cause for a disorder or disease, or allows predicting that a disorder or disease may manifest itself in an organism the sample was obtained from. Making the correct diagnosis based on the detection of a given target nucleic acid or target microorganism allows a physician to draw conclusions as to the correct preventive and/or creative steps, for example treatment with certain medicaments, et cetera.

As used herein, the term "animal" relates to its common meaning in the field, and comprises all types of animals, for example reptiles, amphibians, fish, birds, particularly poultry, insects, domestic animals such as cats, dogs, pigs, cows, camels, horses, mammals in general, and humans.

As used herein, the term "organism of interest" refers to microorganisms which may be the source of disorder or disease as well as to organisms from which a sample was obtained from or is obtainable, for example the clinical sample that may be obtainable from an animal. Of course, an organism of interest may also be a plant, and algae, fungus, et cetera.

As used herein, the term "cell" relates to its common meaning in the field.

As used herein, the term "toxic organism" means that the organism may be the reason for poisoning, including microorganism that produce certain toxins, for example those belonging to the streptococci, viruses producing factors that are haemolytic or otherwise perturb the healthy physiology of an organism, but also multicellular organisms, such as parasites, worms, animals, for example snakes, et cetera.

As used herein, the term "forensic" relates to or denotes the application of scientific methods and techniques to the investigation of crime.

As used herein, the terms "formulation" or "composition" refer to a mixture comprising different chemical compounds, which may be excipients, enzymes, buffers, solvents, nucleic acids or building blocks thereof, sugars, preservatives, salts, etc.

As used herein, the term "environmental sample" refers to a sample which is not a clinical sample, but may be obtained or obtainable from any given environment, for example samples taken from stables comprising domestic animals, restaurants, food, feed, abattoirs, supermarkets, water sources, cantines, surfaces of laboratory devices, et cetera.

As used herein, the term "extracorporeal environment" means that the environment is not part of the body of an animal, plant, or a surface of any of these.

As used herein, the term "hybridizing" refers to the chemical interaction between nucleic acids and has the commonly accepted meaning in the art.

As used herein, the term "kit" refers to a collection of parts that are put together as an entity to be stored, marketed or shipped and often comprises containers comprising compounds of interest, for example different chemical compounds, solutions, buffers, et cetera. These compounds may for example be the controls according to the present invention in a given container, but also polymerase is or mixtures comprising the same, solvents for the reconstitution of freeze-dried/lyophilizated controls and optionally formulations comprising the same, often standards, positive controls, instructions for use, sample collection devices, labware, etc.

As used herein, the term "sample" or "sample of interest" refers to the commonly accepted meaning, and is often a solid or liquid part derived from an organism or from an extracorporeal environment, particularly, it may be a forensic sample or a clinical sample that is suspected to contain a nucleic acid of interest.

According to the present invention, the controls may be encapsulated or adsorbed nucleic acids. These may be used as in-process controls in molecular diagnostic applications. However, it is also possible to spike-in the controls into samples to be analyzed, e.g., clinical samples.

Previously, positive in-process controls were often non-packed ("free") nucleic acid molecules having the desired target sequence often added to nucleic acids previously extracted from a given sample prior to analysis. In particular for regulatory processes it is often important to directly add (or "spike-in") the positive in-process controls to the sample, i.e. prior to the extraction of nucleic acids for the subsequent manipulation steps, such as amplification of nucleic acids, etc..

Due to these previously used protocols two main problems can arise. First, the pure previously prepared positive IP controls (as free NA) are not identical with the actual analyte, for example, a virus particle or a cell. Accordingly, failures to properly lyse and release the nucleic acids are not accurately reflected when "free" nucleic acids are used. Further, free nucleic acids that are directly added to a sample as controls prior to subsequent manipulation steps are extremely sensitive to digestion by nucleases that are almost inevitably contained in the sample to be analyzed. In a worst case scenario and depending on the type of sample (but also depending on the individual sample) this may result in complete failure of the control function. Accordingly, appropriate protection of an in-process control nucleic acid is important. Further, it may be important to also provide a structure of an in-process control that resembles the real-life situation to a favorable extent as, for example in clinical samples, the nucleic acids are often not freely accessible in the sample medium, but generally form part of a higher order structure, e.g. in a cell or microorganism from which the nucleic acids are usually extracted after lysis and purification steps. In other words, preferably the in-process control nucleic acids should be released from a compartment to resemble the situation found in a sample during its processing.

Attempts have been made to approach the real-life situation with in-process controls. For example, nucleic acids have been directly protected using so-called armored RNA (Asuragen; Ref. 1), or by packaging of the nucleic acids in plant viruses (Ref. 2) or lentiviral particles (Ref. 3). These attempts have considerable disadvantages. For example, viral particles have a size limitation of the nucleic acids that may be packaged. Further, production and packaging of the nucleic acids can be extremely time-consuming and costly. There are also limitations regarding the type of nucleic acids that can be packaged (DNA or RNA). These disadvantages are overcome when the FPC's of the present invention are used.

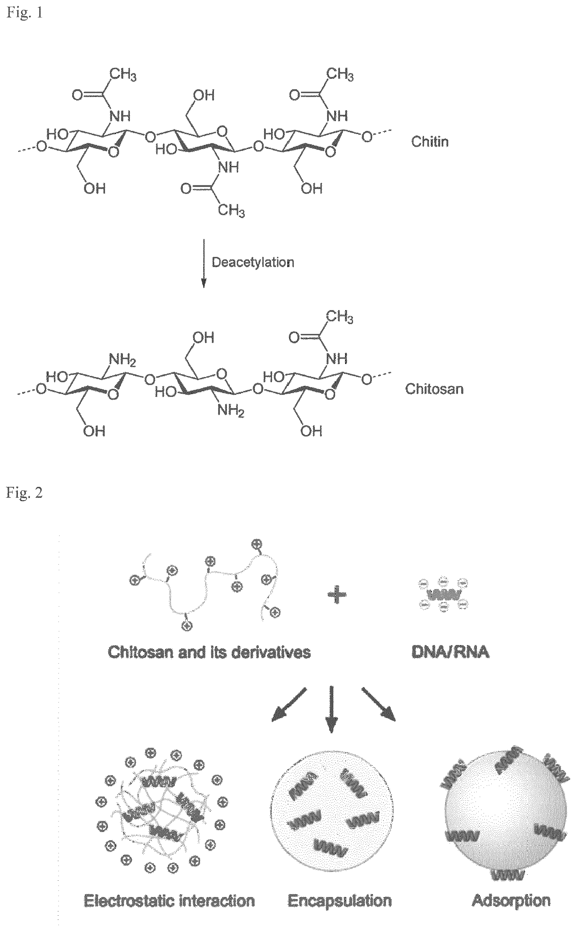

Therefore, according to the present invention, the controls comprise at least partially deacetylated biopolymers, in particular at least partially deacetylated chitin, i.e. chitosan (reference is made to figure 1 which discloses the structure of chitosan) This natural polysaccharide in partly or fully deactylated form may form a highly soluble polycation at a pH value around <6.3.

Due to its unique polycationic properties (the degree of polycationicity can be precisely adjusted by the degree of deacetylation), chitosan had been used for a long time for the complexing of polyanions.

Apart from polyanions such as alginate, pectin, hyaluronic acid, carboxymethyl cellulose, or polyphosphate, nucleic acids had been used for complexation. Increased knowledge of the chitosan molecule's physio-chemical properties (e.g., the degree of polymerization = chain length, degree of acetylation = DA; pattern of acetylation = PA) allowed for the production of nanostructures or nanoparticles. Nanoparticles may be spherical forms with a defined average diameter, e. g., between 10 to 1000 nm, for example 50 to 750 nm, particularly 100 to 500, more particularly 100 to 400 nm).

In the production of nanoparticles at least two components of opposite charge are used. Chitosans may interact with nucleic acids in essentially three different ways (Fig. 2). Irrespective of the interaction with nucleic acids, essentially two different types of nanoparticles exist. First, there are compact nanospheres as shown in Fig. 2, but also nanocapsules which surround emulsified lipid droplets (the latter are often used as medicament carriers, e.g. for the delivery of insulin).

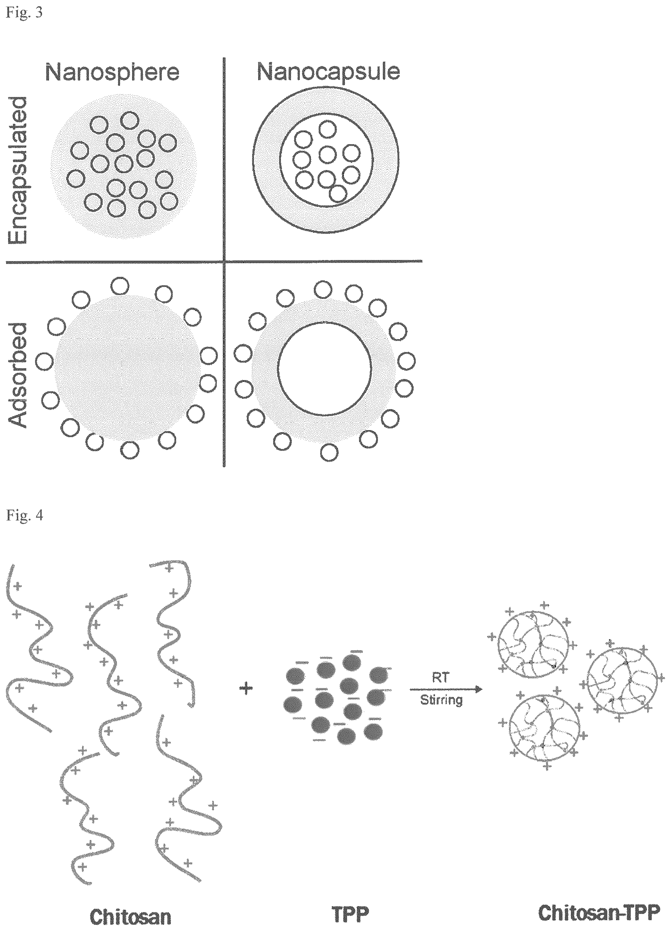

For the interaction with nucleic acids, essentially four different types of structures exist, which are represented in Figure 3 all of which can be used as FPCs of the present invention. In particular embodiments, nanocapsules or nanospheres with adsorbed nucleic acids are provided. The production of these is particularly simply, because "non-loaded" nanoparticles may be produced in larger quantities and then aliquoted to be charged with nucleic acids. This type of loading is technically less demanding and minimizes differences between different charges due to the possibility of providing a large batch of loaded nanoparticles.

In the present invention, nanospheres as well as nanocapsules have been tested in loaded and non-loaded form (Figure 3, right column). Without being loaded, both types of nanoparticles, i.e. nanospheres and nanocapsules did not have an influence on a PCR upon direct spiking in to the PCR reaction mixture and also did not have an influence on the extraction process of nucleic acids upon addition thereof to the sample. Accordingly, it has been shown that nanoparticles do not have a negative influence on the entire process of the sample preparation and purification of nucleic acids as well as to the detection of nucleic acids in PCR, in particular in real-time PCR.

Further nanoparticles (nanocapsules and nanospheres) were loaded with DNA that is specific for parvovirus B19. The loaded nanoparticles were added to samples before nucleic acid extraction using a standard extraction process (AltoStar Purification Kit 1.5). Subsequently the obtained nucleic acids were used in a real-time PCR using a parvovirus B19 specific PCR. In this example the loaded nanocapsules showed very good results, which are comparable with the results obtained upon extraction of a conventional positive control. The loaded nanospheres showed slightly less positive applicability, but are still surprisingly well-suited for the inventive purposes.

The loaded nanocapsules were successfully calibrated against the established parvovirus B19 WHO standard (3rd WHO International Standard for Parvovirus B19 for Nucleic Acid Amplification Techniques, NIBSC code: 12/208). In subsequently performed experiments, the calibrated particles were diluted in plasma and were correctly quantified using real-time PCR. Conventionally, non-protected DNA is degraded in plasma within a few minutes so that samples cannot be detected using real-time PCR. This could be shown in the experiments using parallel analysed sample of the same plasma with non-protected DNA. Initial investigations of the stability have also shown that nanocapsules according to the present invention which were diluted in plasma could be correctly quantified upon storage for 24 weeks at 2 to 8°C and at -25°C to -15°C as well as after three freeze-thaw cycles of stored nanocapsules in samples that were stored at -25°C to -15°C. These experimental results indicate that the nanoparticles are a) very stable and b) that they can protect the nucleic acid from degradation by nuclease over an extended period of time and harsh conditions, which is a huge advantage when compared to conventional controls.

Further, loaded nanocapsules were added to different types of samples, i.e. plasma, serum, universal transport medium (UTM), urine, stool and whole blood. Nucleic acids were extracted from the samples and analysed using real-time PCR. It could be shown that samples that were spiked with nanocapsules provided results that were comparable to samples tested as controls which contained the respective concentration of the parvovirus B19 WHO standard (Figure 4).

### Experiments

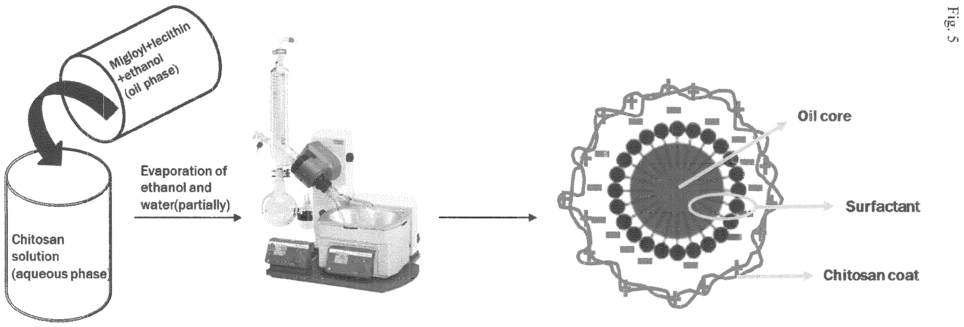

For the generation of chitosan nanospheres the method of ionic gelation with tri polyphosphate (TPP) was used (Figure 4). Briefly, chitosans of different DAs were dispersed in water together with acetic acid, stirred at room temperature and a TTP stock solution was added dropwise (Scientific Reports, volume 8, Article number: 4695 (2018)). The particles formed spontaneously by ionic gelation. For the generation of chitosan nanocapsules the solvent displacement technique was used by forming a lecithin stabilized nanoemulsion (Figure 5). Briefly, Migloyl, lecithin and ethanol were combined to form an organic phase, which was poured in an aqueous phase composed of a chitosan solution (Nanomaterials 2018, 8(10), 846)). The nanocapsules formed spontaneously due to the organic solvents diffusion and Marangoni effects of the organic phase. The quality control of the generated particles (nanospheres and nanocapsules) in different aspects (size, charge, size distribution [polydispersity index]) was determined with a Zetasizer Nano ZS™ instrument (Malvern Instruments, UK).

### Testing of unloaded nanosystems

Aim of this study was to examine whether chitosan nanospheres and/or nanocapsules influence the nucleic acid extraction process and/or the PCR mediated amplification of target RNA/DNA. Up to 400 particles/µl were either added to HBV and HCV positive plasma samples prior to extraction or directly to the PCR reaction. Purification and amplification/detection were performed using the AltoStar Purification Kit 1.5 and the AltoStar HBV PCR Kit 1.5 and AltoStar HCV RT-PCR Kit 1.5. Compared to the reference without addition of particles the quantification results for the high positive (HP) as well as the detection rate for the sensitivity controls (SC) were in both cases not affected by the addition of up to 400 particles/µl. All sensitivity controls were positive and mean quantification results did not differ by more than 0.08 log. Taken together, chitosan nanosystems did not influence the nucleic acid extraction process and/or the PCR mediated amplification of target RNA/DNA. The results are summarized in table 1.

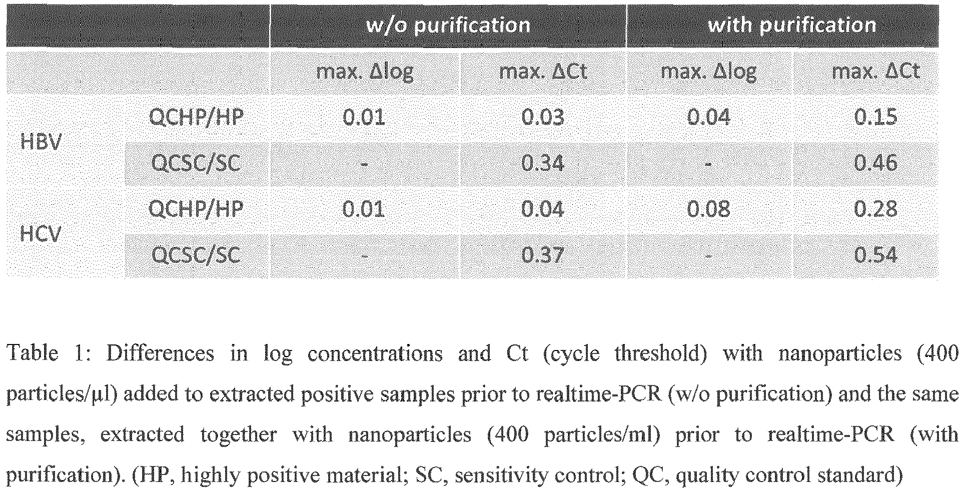

### Testing of Parvovirus B 19 target sequence loaded nanosystems

Aim of this study was to examine the functionality and stability (stress test) of chitosan nanospheres and/or nanocapsules "loaded" with a Parvovirus B19 specific DNA fragment stored in human EDTA plasma. Loaded nanospheres and nanocapsules were diluted in Parvovirus B19 negative human EDTA plasma and extracted prior to amplification using the AltoStar Purification Kit 1.5. Whereas nanospheres with a load ratio of 1:1 (1 particle with 1 molecule of target DNA sequence) gave no signal in realtime-PCR, nanocapsules with a load ratio of 1:1 gave a good signal. This signal was comparable with twice the amount of nanospheres, which were loaded with 10 times more target DNA molecules. Taken together, Parvovirus B19 DNA adsorbed on both types of nanosystems can be extracted from human EDTA plasma and amplified by realtime-PCR.

Nanospheres and/or nanocapsules "loaded" with a Parvovirus B19 specific DNA fragment were diluted in Parvovirus B19 negative human EDTA plasma and calibrated against the 3^{rd} WHO International Standard for Parvovirus B19 for Nucleic Acid Amplification Techniques (NIBSC code: 12/208). Based on these results IU/ml (international Units per ml) concentrations were assigned to the different nanosystems.

For the stress test loaded nanoparticles and/or nanocapsules were diluted to a concentration of 1x10³ IU/ml in human EDTA-plasma and stored for three days at different temperatures. In addition the loaded nanoparticles and/or nanocapsules were subjected to 1 to 3 freeze/thaw cycles with intermittent storage at -25 to -15°C. After storage for 3 days sample were extracted using the AltoStar Purification Kit 1.5 and analysed using a Parvovirus B19 specific realtime-PCR. Results were compared to freshly prepared dilutions of loaded nanoparticles and/or nanocapsules in human EDTA-plasma.

The results are summarized in table 2. For the the 1:1 loaded nanocapsules the results were always comparable to the freshly prepared sample (mean quantification difference from the nominal value was ≤ 0.17 log), even for the freeze/thaw sample. The results for the loaded 1:10 nanospheres were inconsistent with the reference showing the greatest difference from the nominal value (0.64 log).

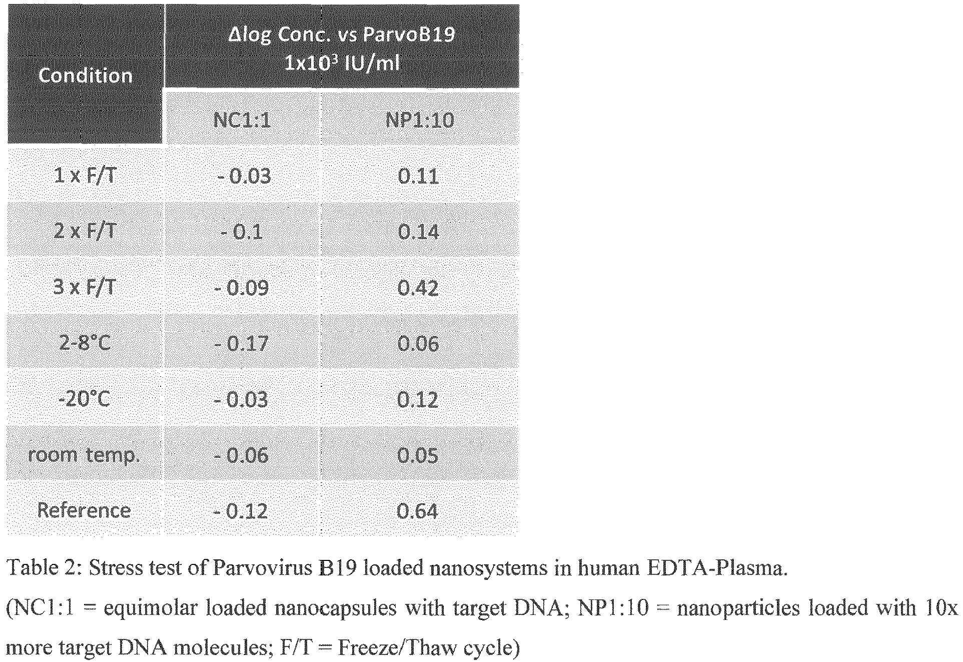

Those results were generated in a proof-of-principle study with only one kind of nanocapsule and nanoparticle with only one kind of chitosan (in terms of DP, DA and PA). It may well be possible, that other chitosan formulations have a more positive effect on our purpose of a full process control and thus may also render chitosan nanospheres a good performing candidate.

### Long-term stability test

For a long term stability test loaded nanocapsules and nanoparticles were each diluted in human EDTA-Plasma to 1000 IU/ml and 250 IU/ml of Parvovirus B19 DNA, extracted directly and subjected to realtime-PCR and extracted and tested after a storage at room temperature, 2-8°C, -20°C for 1, 2, 4, 6, 8, 12, and 24 weeks.

Whereas the nanoparticles already showed a significant standard deviation in Ct-values after 1 week and the Ct-values got worse beyond the tolerable limit after storage at 2-8°C and -20°C after 8 weeks. The nanocapsules remained stable (in terms of quantification results for the 1000 IU/ml (max. Δlog 0.32) and detection rate of 100% for the 250 IU/ml) even after 24 weeks of storage at 4-8°C and - 20°C.

### Matrix stability tests

Parvovirus B19 loaded nanocapsules or the 3^{rd} WHO International Standard for Parvovirus B19 for Nucleic Acid Amplification Techniques (NIBSC code: 12/208) were diluted to the same concentration (1000 IU/ml) in different commonly used sample matrices (plasma, serum, viral transport medium, urine, stool and whole blood), nucleic acids were extracted using the AltoStar Purification Kit 1.5 and samples were analysed using a Parvovirus B19 specific realtime-PCR (table 3).

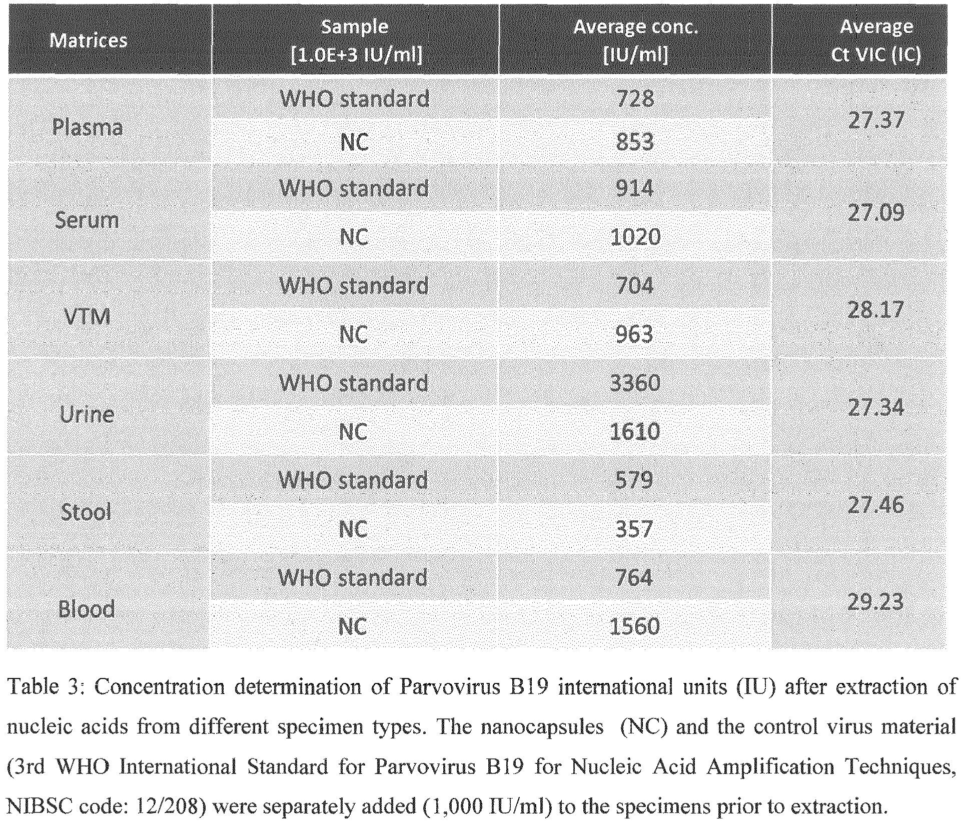

Quantification results for the diluted nanocapsules from all matrices were comparable (max. Δlog 0.3) to the 3^{rd} WHO International Standard for Parvovirus B19 and within 0.5 log compared to the expected value (1000 IU/ml).

## Claims

1. A control for nucleic acid preparation and/or detection methods comprising at least one polycationic particle and a nucleic acid.

2. The control for nucleic acid preparation and/or detection methods according to claim 1, wherein the polycationic particle comprises a polycationic polysaccharide, a polycationic amino acid, particularly polylysine, or derivative of any of these.

3. The control for nucleic acid preparation and/or detection methods according to claims 1 or 2, wherein polycationic polysaccharide comprises at least partially deacetylated N-acetyl-D-glucosamine (GlcNAc, A) residues and/or a derivative thereof.

4. The control for nucleic acid preparation and/or detection methods according to any one of the preceding claims 1 to 3, wherein the polycationic polysaccharide comprises at least partically deacetylated N-acetyl-D-glucosamine (GlcNAc, A) residues co-polymerized with D-glucosamine (GlcN, D) and/or a derivative thereof.

5. The control for nucleic acid preparation and/or detection methods according to any one of the preceding claims 1 to 4, wherein the polycationic polysaccharide comprises chitosan and/or a derivative thereof.

6. The control for nucleic acid preparation and/or detection methods according to any one of the preceding claims 1 to 5, wherein the polycationic polysaccharide and the nucleic acid are present in form of an adsorbed nanoparticle, an adsorbed nanosystem an adsorbed nanosphere or in form of an adsorbed nanocapsule, or wherein the nucleic acid is present in a polycationic polysaccharide nanoparticle, a polycationic polysaccharide nanosystem an adsorbed polycationic polysaccharide nanosphere or in form of an adsorbed polycationic polysaccharide nanocapsule.

7. Use of the control for nucleic acid preparation and/or detection methods according to any of the preceding claims 1 to 6, in a nucleic acid detection method.

8. Use according to claim 7, wherein the nucleic acid detection method is a diagnostic or in a forensic method or in a step of a diagnostic or a forensic method.

9. Use according to any one of claims 7 and 8, wherein the nucleic acid is present in an extracorporeal environment and is indicative of the presence of an organism of interest, an infectious agent, a parasite, a toxic organism, from a microorganism, a bacteria, a virus, a fungus, a parasite, an archaea, or an eukaryote.

10. A formulation or a composition comprising a control according to anyone of the preceding claims 1 to 6.

11. The formulation or composition according to claim 10 or the control according to any one of claims 1 to 6, wherein said formulation or composition or said control is in a lyophilized state.

12. A method for the detection of a target nucleic acid, comprising the step of adding the control according to anyone of claims 1 to 6, or a formulation or a composition according to anyone of claims 10 to 11 to a sample.

13. The method according to claim 12, wherein said method comprises the following steps:
e) adding the control according to any one of claims 1 to 6 or a formulation or a composition according to anyone of claims 10 or 11 to a sample to form a mixture,
f) obtaining nucleic acids from the mixture obtained in step a),
g) performing an amplification reaction using oligonucleotides specifically hybridizing with at least one target nucleic acid and/or oligonucleotides specifically hybridizing with the control nucleic acid,
h) measuring / detecting the amplification product obtained in step c).

14. A kit of parts comprising at least one reagent for the sampling of nucleic acids and/or for the extraction of nucleic acids and/or for the amplification of nucleic acids and/or for the detection of nucleic acids, and a control or a formulation or a composition comprising a control according to any of the preceding claims 1 to 6 and/or 10 or 11, and optionally instructions for use.

15. A point-of-care device comprising a control for nucleic acid preparation and/or detection methods according to any of the preceding claims 1 to 6, or a formulation or composition according to any one of claims 10 or 11.
